Europäisches Patentamt

European Patent Office

Office européen des brevets

(11) Publication number: **0 535 110 B1**

(12) **EUROPEAN PATENT SPECIFICATION**

(45) Date of publication of patent specification: **14.12.94**  (51) Int. Cl.5: **C12N 9/52**, C11D 3/386

(21) Application number: **91911972.7**

(22) Date of filing: **14.06.91**

(86) International application number:
**PCT/DK91/00160**

(87) International publication number:
**WO 91/19792 (26.12.91 91/29)**

(54) **THERMOSTABLE PROTEASE FROM THERMOCOCCUS.**

(30) Priority: **15.06.90 DK 1458/90**

(43) Date of publication of application:
**07.04.93 Bulletin 93/14**

(45) Publication of the grant of the patent:
**14.12.94 Bulletin 94/50**

(84) Designated Contracting States:
**AT BE CH DE DK ES FR GB GR IT LI LU NL SE**

(56) References cited:
**WO-A-90/10072**
**US-A- 3 796 635**
**US-A- 4 480 036**

**Chemical Abstracts, vol. 108, no. 5, 1 Febr. 1988 (Columbus, Ohio, US) Cowan Don A. et al.**

**Chemical Abstracts, vol. 114, no. 13, 1 April 1991, /Columbus, Ohio, US), Zamost Bruce L. et al. abstr. 117332y, & J. Ind. Microbiol.. 1990, 5 (5), 304-312**

**Patent Abstracts of Japan, vol. 12, no. 472, C551 abstract of JP 63-192387, publ 1988-08-09 (TOSOH CORP)**

**Chemical Abstracts, vol. 82, no. 1, 6 Jan. 1975, (Columbus, Ohio, US), s. p. 244, abstract 2625z,**

**Chemical Abstracts, vol. 112, no. 1, 1 Jan. 1990, (Columbus, Ohio, US), s. p. 618, abstract 6098r, & JP,, 137290 ((TOYOBO CO. LTD.)) 1989**

(73) Proprietor: **NOVO NORDISK A/S**
**Novo Allé**
**DK-2880 Bagsvaerd (DK)**

(72) Inventor: **ANTRANIKIAN, Garabed**
**Dahlienweg 20**
**D-2105 Seevetal 1-Hittfeld (DE)**
Inventor: **KLINGEBERG, Michael**
**Südstrasse 8a**
**D-3212 Gronau (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.09/3.3.3)

## Description

## TECHNICAL FIELD

This invention is within the field of thermostable proteases. More specifically, the present invention relates to novel thermostable proteases, a process for the preparation of these enzymes, and detergent compositions comprising these enzymes.

## BACKGROUND ART

Hyperthermophilic archaebacteria have been isolated from solfataric and submarine hydrothermal systems (vide e.g. Kelly, R.M. & Deming, J.W.; Biotech. Progress, 4, 47-62 (1988)). It has been presumed that members of Thermococcus contain heat stable proteases and amylases (Stetter, K.O.; J. Chem. Technol. Biotechnol., 42(4), 315-317 (1988)). However, proteases from Thermococcus have not formerly been isolated or investigated.

## SUMMARY OF THE INVENTION

Within the scope of the present invention novel enzymes that show extraordinary thermostability as well as thermoactivity are provided. Accordingly, in its first aspect, the present invention provides a protease that is characterized by having pH optimum in the range 6.0 to 10.0, temperature optimum in the range 75° to 100°C, and immunochemical properties identical to those of the protease derived from Thermococcus celer, DSM No. 2476; Thermococcus sp. AN1, DSM No. 2770; Thermococcus stetteri, DSM No. 5262; or Thermococcus litoralis, DSM No. 5474.

In another aspect, the invention provides a protease having pH optimum in the range of from pH 6.0 to 10.0, temperature optimum in the range of from 75 to 100°C, and being derived from a strain of the species Thermococcus celer, Thermococcus sp.AN1, Thermococcus stetteri, or Thermococcus litoralis.

In yet another aspect, the present invention provides a process for the preparation of these proteases, which process comprises cultivation of a protease producing strain of Thermococcus in a suitable nutrient medium, containing carbon and nitrogen sources and inorganic salts, followed by recovery of the desired enzyme.

In preferred embodiments of this process, a strain of Thermococcus celer; a strain of Thermococcus sp. AN1; a strain of Thermococcus stetteri; or a strain of Thermococcus litoralis is cultivated.

In further preferred embodiments of this process Thermococcus celer, DSM No. 2476; Thermococcus sp. AN1, DSM No. 2770; Thermococcus stetteri, DSM No. 5262; or Thermococcus litoralis, DSM No. 5474; or mutants or variants thereof, is cultivated.

In further aspects, the invention provides a proteolytic detergent additive and a detergent composition, both comprising a protease of the invention.

## BRIEF DESCRIPTION OF DRAWINGS

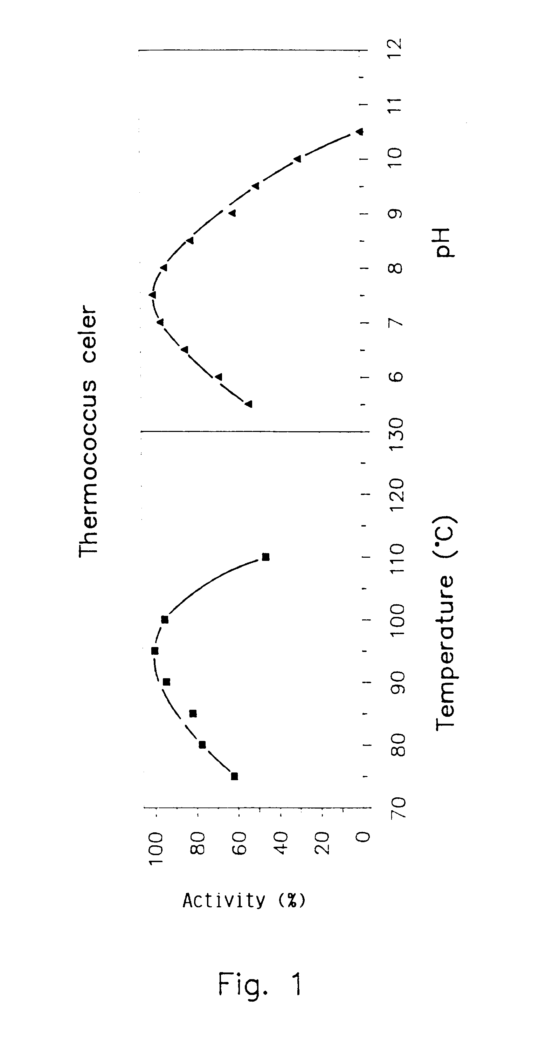

The present invention is further illustrated by reference to the accompanying drawings, in which the Figs. 1-4 show the relation between the enzymatic activity of proteases obtained from Thermococcus celer; Thermococcus sp. AN1; Thermococcus stetteri; Thermococcus litoralis; and temperature and pH, respectively.

## DETAILED DISCLOSURE OF THE INVENTION

Growth experiments with Thermococcus have now shown that these organisms secrete extremely thermostable and thermoactive protein hydrolysing enzymes. These enzymes possess proteolytic activity under extreme conditions. The properties of Thermococcus are demonstrated by Thermococcus celer, Thermococcus sp. AN1, Thermococcus stetteri, and Thermococcus litoralis. A strain of Thermococcus celer is available from DSM, No. 2576, a strain of Thermococcus sp. AN1 is available from DSM, No. 2770, a strain of Thermococcus stetteri is available from DSM, No. 5262, and a strain of Thermococcus litoralis is available from DSM, No. 5474.

As appears from the figures, the proteases obtainable from Thermococcus can be characterized by a pH optimum in the range of from 6.0 to 10.0, and a temperature optimum in the range of from 75° to 100°C.

Further, it appears from Fig. 1 that the protease obtainable from Thermococcus celer is active in a broad temperature range, namely at temperatures of from below 75°C to above 110°C, and in a pH range of from below 5.5 to 10.5. The temperature optimum is between 90° and 100°C, more specifically around 95°C. 60% enzymatic activity are detected at 75°C, and 40% enzymatic activity are detected at 110°C. The enzyme has a pH optimum in the range of from 6.5 to 8.5, more specifically of from 7.0 to 8.0 (around 7.5). Considerable enzyme activity is still detected at pH 5.5 and 10.0, respectively.

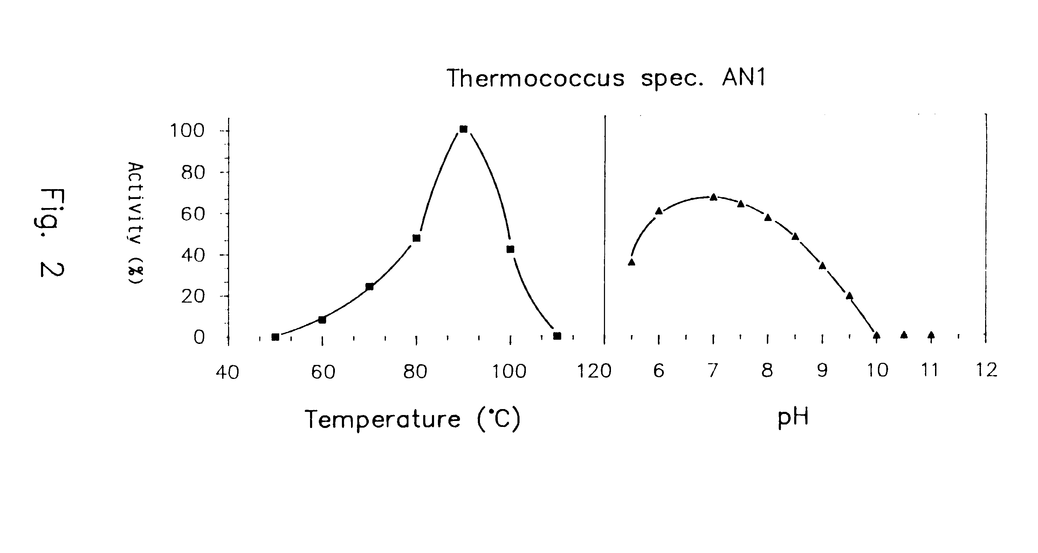

From Fig. 2 it appears that the protease obtainable from Thermococcus sp. AN1 is active in a temperature range of from 50 to 110°C, and in a pH range of from below 5.5 to 10. The protease has a temperature optimum in the range of from 80° to 100°C, more specifically around 90°C, and a pH optimum in the range of from 6.0 to 8.0, more specifically around 7.0.

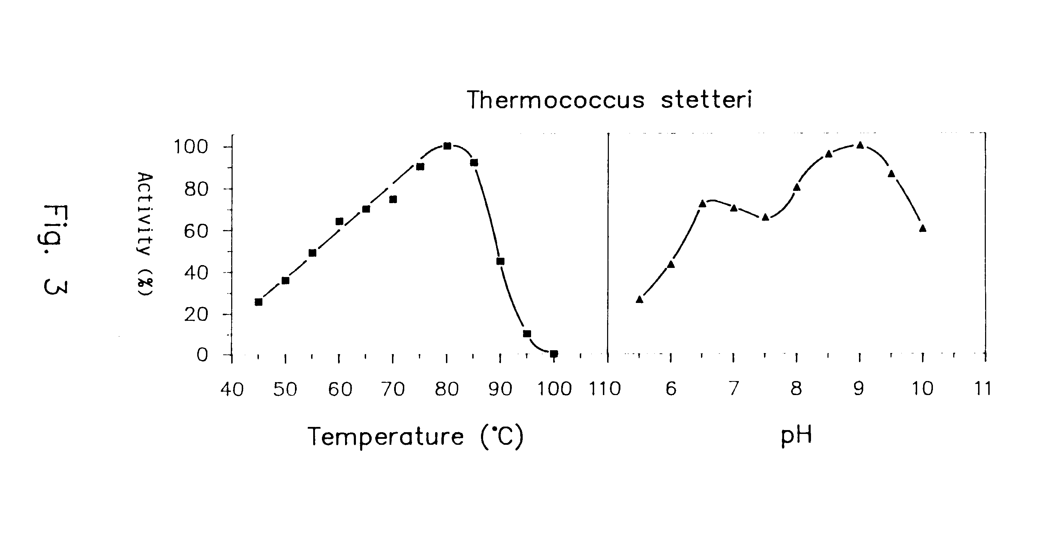

From Fig. 3 it appears that the protease obtainable from Thermococcus stetteri is active in a temperature range of from below 45°C to 100°C, and in a pH range of from below 5.5 to above 10. The protease has a temperature optimum in the range of from 75° to 85°C, more specifically around 80°C, and a pH optimum in the range of from pH 8 to 10, more specifically around pH 9.0.

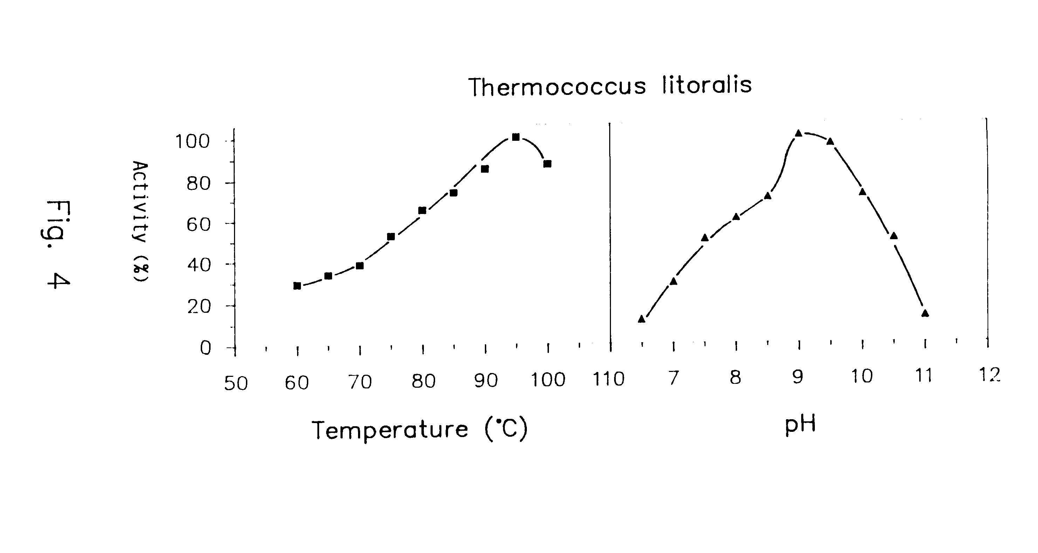

From Fig. 4 it appears that the protease obtainable from Thermococcus litoralis is active in a temperature range of from below 60°C to above 100°C, and in a pH range of from below 6.5 to above 11. The protease has a temperature optimum in the range of from 90° to 100°C, more specifically around 95°C, and a pH optimum in the range of from pH 8 to 10, more specifically around pH 9.0.

In table 1 some properties of the proteases from Thermococcus sp. are shown.

Table 1

|  | T. celer | T. AN 1 | T. stett. | T. litor. |
|---|---|---|---|---|
| pH optimum | 7.5 | 7.0 | 9.0 | 9.0 |
| temperature optimum | 95 | 90 | 80 | 95 |
| type | serine | serine | serine | serine |
| substrate specificity: |  |  |  |  |
| Z-DL-Arg-pNA | - | + | + | + |
| Suc-Ala-Ala-Pro-Phe-pNA | + | + | + | + |
| Z-DL-Lys-pNA | - | - | + | + |
| Z-Gly-Pro-pNA | - | + | - | + |
| D-Phe-Pip-Arg-pNA | + | + | + | + |
| D-Val-L-Leu-Lys-pNA | + | - | + | - |
| Suc = Succinyl<br>pNA = p-nitroanilide<br>Pip = piperazine |  |  |  |  |

The immunochemical properties can be determined immunologically by cross-reaction identity tests. The identity tests can be performed by the well-known Ouchterlony double immunodiffusion procedure or by tandem crossed immunoelectrophoresis according to N. H. Axelsen; Handbook of Immunoprecipitation-in-Gel Techniques; Blackwell Scientific Publications (1983), chapters 5 and 14. The terms "antigenic identity" and "partial antigenic identity" are described in the same book, chapters 5, 19 and 20.

Preparation of the proteases

The proteases according to the present invention can be prepared by cultivation of a protease producing strain of Thermococcus in a suitable nutrient medium, containing carbon and nitrogen sources and inorganic salts, followed by recovery of the desired enzyme, by methods known in the art.

The protease according to the present invention can also be prepared by recombinant DNA-technology.

Detergent compositions

Due to the unique properties of the proteases according to the present invention, these enzymes are of great interest for industrial applications, e.g. for use in the detergent industry.

The detergent composition of the invention may comprise one or more surfactants, which may be of an anionic, non-ionic, cat-ionic, amphoteric or zwitter-ionic type, or a mixture of these. Typical examples of anionic surfactants are linear alkyl benzene sulfonates (LAS); alkyl sulfates (AS); alpha olefin sulfonates (AOS); alcohol ethoxy sulfates (AES) and alkali metal salts of natural fatty acids. Examples of non-ionic surfactants are alkyl polyethylene glycol ethers; nonylphenol polyethylene glycol ethers; fatty acids esters of sucrose and glucose; and esters of polyethoxylated alkyl glucoside.

The detergent composition of the invention may also contain other detergent ingredients known in the art such as builders, bleaching agents, bleach activators, anti-corrosion agents, sequestering agents, anti soil-redeposition agents, perfumes, stabilizers for the enzymes and bleaching agents, formulations aids, optical brighteners, foam boosters, chelating agents, fillers, fabric softeners, etc. The detergent composition of the invention may be formulated substantially as described in J. Falbe [Falbe, J.; Surfactants in Consumer Products. Theory, Technology and Application; Springer Verlag 1987, vide in particular the section entitled "Frame formulations for liquid/powder heavy-duty detergents"].

It is at present contemplated that the detergent composition of the invention may contain the enzyme preparation in an amount corresponding to 0.0005-0.5 CPU of the proteolytic enzyme per litre of washing liquor.

The detergent compositions of the invention can be formulated in any convenient form, such as powders, liquids, etc.

The detergent composition of the invention may advantageously include one or more other enzymes, e.g. lipases; amylases; cellulases; and/or peroxidases, conventionally included in detergent compositions.

The protease of the invention may be included in a detergent composition by adding separate additives containing the detergent protease, or by adding a combined additive comprising different detergent enzymes.

The additive of the invention can be formulated e.g. as granulates, liquids, slurries, etc. Preferred detergent additive formulations are non-dusting granulates, liquids, in particular stabilized liquids, slurries, or protected enzymes. Dust free granulates may be produced according to e.g. GB 1,362,365 or US 4,106,991, and may optionally be coated by methods known in the art. The detergent enzymes may be mixed before or after granulation. Liquid enzyme preparations may, for instance, be stabilized by adding a polyol such as e.g. propylene glycol; a sugar or sugar alcohol; lactic acid or boric acid, according to established methods. Other enzyme stabilizers are well known in the art. Protected enzymes may be prepared according to the method disclosed in EP 238,216.

The following examples further illustrate the present invention.

**EXAMPLE 1**

Cultivation of Thermococcus celer

Thermococcus celer, DSM No. 2476, was cultivated in a nutrient medium containing the following components (per 1 litre):

| NaCl | 40.00 g |
|---|---|
| $(NH_4)_2 SO_4$ | 1.30 g |
| $KH_2 PO_4$ | 0.28 g |
| $MgSO_4 \cdot 7H_2O$ | 0.25 g |
| $CaCl_2 \cdot 2 H_2O$ | 0.07 g |
| $FeCl_3 \cdot 6H_2O$ | 2.000 mg |
| $MnCl_2 \cdot 4H_2O$ | 1.800 mg |
| $Na_2 B_4 O_7 \cdot 10H_2O$ | 4.500 mg |
| $ZnSO_4 \cdot 7H_2O$ | 0.220 mg |
| $CuCl_2 \cdot 2H_2O$ | 0.050 mg |
| $Na_2 MoO_4 \cdot 2H_2O$ | 0.030 mg |
| $VOSO_4 \cdot 5H_2O$ | 0.038 mg |
| $CoSO_4$ | 0.010 mg |
| Yeast extract | 1.00 g |
| Peptone | 1.00 g |
| Resazurin | 1.000 mg |
| Sulphur (powdered) | 5.00 g |
| $Na_2 S \cdot 9H_2O$ | 0.50 g |
| Adjust pH to pH 5.8 | |

The medium without sodium sulphide and sulphur was boiled for 20 minutes, cooled on ice and dispensed under $N_2$ atmosphere. The medium was then filled under $N_2$ atmosphere into 100-ml vials containing sulphur. For sterilization the medium was heated to 100°C for 1 hour on each of 3 successive days.

Before inoculation the medium was reduced by adding 10 ml/l of sterile neutral sodium sulphide (5% solution). The medium was inoculated with 10% of a grown preculture and finally incubated at 88°C for 24-36 hours.

Assay for proteolytic activity

The enzymatic reaction was conducted in a 50 mM of phosphate buffer, pH 7.0, containing 0.25% of casein (Hammarsten, Serva, Heidelberg, FRG). The reaction was initiated by the addition of 250 $\mu$l of cell suspension to 2250 $\mu$l assay mixture. Samples (500 $\mu$l each) were taken after incubation at 90°C for 30, 60, 90, and 120 min. The reaction was stopped by cooling the samples on ice and by the addition of 500 $\mu$l of trichloroacetic acid (10% solution). The mixture was allowed to stand at room temperature for 30 min. and then centrifuged for 10 min. at 12000 rpm. The absorbance of the supernatant was measured at 280 nm against a blank. 1 U of enzyme is defined as the amount of enzyme which liberates 1$\mu$mol of tyrosine per min.

Characterization of the protease

In order to study the effect of pH and temperature on enzyme activity a buffer mixture which was composed of 20 mM MES (2-[N-Morpholino]ethanesulfonic acid), 20 mM HEPES (N-2-hydroxyethylpiperazine-N'-2-ethanesulfonic acid) and 20 mM Glycine was used. The pH value was varied from 5.5 to 11.0 in increments of 0.5, and the temperature was varied from 75 to 110°C. Casein (0.25%) was used as a substrate.

As shown in Fig. 1, the protease from T. celer was active in a broad temperature and pH range. 60% and 40% of enzyme activity was still detected at 75°C and 110°C, respectively; the temperature optimum being 90 to 100°C. The enzyme had a pH optimum of around 7.5, and considerable activity was also detected at pH 5 and 10.

**EXAMPLE 2**

Cultivation of Thermococcus sp. AN1

Thermococcus sp. AN1, DSM No. 2770, was cultivated in a nutrient medium containing the following components (per litre):

| Trypticase (BBL) | 10.0 g |
|---|---|
| $KH_2PO_4$ | 1.5 g |
| NaCl | 2.5 g |
| Sulphur (powdered) | 8.0 g |
| $Na_2S \cdot 9H_2O$ | 0.5 g |
| Resazurin | 1.0 mg |
| Adjust pH to 7.3 | |

The medium without sodium sulphide and sulphur was boiled for 20 minutes, cooled on ice and dispensed under $N_2$ atmosphere. The medium was then filled into a 100-ml vial containing sulphur under $N_2$ atmosphere. For sterilization the medium was heated to 100°C for 1 hour on each of 3 successive days.

Before inoculation with 10% of a grown preculture the medium was reduced by adding 10 ml/l of sterile neutral sodium sulphide (5% solution). Bacteria were cultivated at 75°C for 24-36 hours.

### Assay for proteolytic activity

The enzymatic reaction was conducted in a 50 mM of phosphate buffer, pH 7.0, containing 0.25% of casein (Hammarsten, Serva, Heidelberg, FRG). The reaction was initiated by the addition of 250 $\mu$l of cell suspension to 2250 $\mu$l assay mixture. Samples (500 $\mu$l each) were taken after incubation at 80°C for 30, 60, 90, and 120 min. The reaction was stopped by cooling the samples on ice and by the addition of 500 $\mu$l of trichloroacetic acid (10% solution). The mixture was allowed to stand at room temperature for 30 min. and then centrifuged for 10 min. at 12000 rpm. 1 U of enzyme is defined as the amount of enzyme which liberates 1 $\mu$mol of tyrosine/min.

### Characterization of the protease

Casein (Hammarsten) was dissolved at a concentration of 0.25% in a buffer mixture which was composed of 20 mM MES (2-[N-Morpholino]ethanesulfonic acid), 20 mM HEPES (N-2-hydroxyethylpiperazine-N'-2-ethanesulfonic acid) and 20 mM Glycine at pH-values from 5.5 to 9, and at temperatures from 50 to 110°C.

As shown in Fig. 2, the level of protease activity reached a maximum around pH 7.0. At a pH-value of 5.5 and 9.0 approximately 35% of activity could be detected. Optimum proteolytic activity for the substrate casein occurred at 90°C. Approximately 50% of enzyme activity was measured at 100°C.

### EXAMPLE 3

### Cultivation of Thermococcus stetteri

Thermococcus stetteri, DSM No. 5262, was cultivated in a nutrient medium containing the following components (per litre):

| NaCl | 25.00 g |
|---|---|
| $NH_4Cl$ | 0.33 g |
| $CaCl_2 \cdot 2 H_2O$ | 0.33 g |
| $MgCl_2 \cdot 6 H_2O$ | 0.33 g |
| KCl | 0.33 g |
| $KH_2PO_4$ | 0.33 g |
| Trace element solution (see DSM-medium 320) | 1.0 ml |
| Vitamin solution (see DSM-medium 320) | 10.0 ml |
| Trypticase BBL | 5.00 g |
| Sulphur powdered | 10.00 g |
| Resazurin | 1.00 mg |
| $Na_2S \cdot 9 H_2O$ | 0.50 g |
| pH 5.7 | |

The medium without sodium sulphide and sulphur was boiled for 20 minutes, cooled on ice and dispensed under $N_2$ atmosphere. The medium was then filled into a 100-ml vial containing sulphur under $N_2$ atmosphere. For sterilization the medium was heated to 100°C for 1 hour on each of 3 successive days.

Before inoculation with 10% of a grown preculture the medium was reduced by adding 10 ml/l of sterile neutral sodium sulphide (5% solution). Bacteria were cultivated at 80°C for 24-36 hours.

Assay for proteolytic activity

The assay mixture contained 0.25% casein (Hammarsten) which was dissolved in 50 mM Tris/Glycine buffer, pH 9.0. The reaction was initiated by the addition of 250 $\mu$l enzyme sample to 2350 $\mu$l assay mixture at 80°C. Samples (500 $\mu$l each) were taken after 30, 60, 90, 120 min. The reaction was stopped by cooling on ice and by the addition of 500 ml of trichloroacetic acid (10%). The mixture was allowed to stand at room temperature for about 30 minutes and centrifuged afterwards for 10 minutes at 12,000 r.p.m. The absorbance of the supernatant was determined at 280 nm against a blank. 1 U of enzyme is defined as that amount of enzyme which liberates 1 $\mu$mol of tyrosine per minute under the specified conditions.

Characterization of the protease

Casein (Hammarsten) was dissolved at a concentration of 0.25% in a buffer mixture which was composed of 20 mM MES (2-[N-Morpholino]ethanesulfonic acid), 20 mM HEPES (N-2-hydroxyethylpiperazine-N'-2-ethanesulfonic acid) and 20 mM Glycine at pH-values from 5.5 to 10, and at temperatures from 45 to 100°C.

As shown in Fig. 3, the level of protease activity reached a maximum around pH 9.0. At a pH-value of 5.5 approximately 30% of activity could be detected and at a pH-value of 10.0 approximately 70% of activity could be detected. Optimum proteolytic activity for the substrate casein occurred at 80°C. Approximately 30% of enzyme activity was measured at 45°C.

**EXAMPLE 4**

Cultivation of Thermococcus litoralis

Thermococcus litoralis, DSM No. 5474, was cultivated in a nutrient medium containing the following components (per litre):

| | |
|---|---|
| NaCl | 19.45 g |
| $MgCl_2 \cdot 6 H_2O$ | 12.60 g |
| $Na_2SO_4$ | 3.42 g |
| $CaCl_2 \cdot 2 H_2O$ | 2.38 g |
| KCl | 0.55 g |
| $Na_2CO_3$ | 0.61 g |
| KBr | 0.08 g |
| $SrCl_2$ | 57.2 mg |
| $Na_2HPO_4$ | 10.0 mg |
| Na metasilicate | 4.0 mg |
| NaF | 2.4 mg |
| $KNO_3$ | 1.6 mg |
| Resazurin | 100.0 mg |
| Yeast extract | 1.0 g |
| Bactopeptone | 5.0 g |
| Sulphur powdered | 10.0 g |
| $Na_2S \cdot 9 H_2O$ | 0.5 g |
| ad 1000 ml $H_2O$dest. pH 6.5 | |

The medium without sodium sulphide and sulphur was boiled for 20 minutes, cooled on ice and dispensed under $N_2$ atmosphere. The medium was then filled under $N_2$ atmosphere into 100-ml vials containing sulphur. For sterilization the medium was heated to 100°C for 1 hour on each of 3 successive days.

Before inoculation the medium was reduced by adding 10 ml/l of sterile neutral sodium sulphide (5% solution). The medium was inoculated with 10% of a grown preculture and finally incubated at 85°C for 24-36 hours.

Assay for proteolytic activity

The assay mixture contained 0.25% casein (Hammarsten) which was dissolved in 50 mM Tris/Glycine buffer, pH 9.0. The reaction was initiated by the addition of 250 $\mu$l enzyme sample to 2350 $\mu$l assay mixture at 90°C. Samples (500 $\mu$l each) were taken after 30, 60, 90, 120 min. The reaction was stopped by cooling and by the addition of 500 ml of trichloroacetic acid (10%). The mixture was allowed to stand at room temperature for about 30 minutes and centrifuged afterwards for 10 minutes at 12,000 r.p.m. The absorbance of the supernatant was determined at 280 nm against a blank. 1 U of enzyme is defined as that amount of enzyme which liberates 1 $\mu$mol of tyrosine per minute under the specified conditions.

Characterization of the protease

Casein (Hammarsten) was dissolved at a concentration of 0.25% in a buffer mixture which was composed of 20 mM MES (2-[N-Morpholino]ethanesulfonic acid), 20 mM HEPES (N-2-hydroxyethylpiperazine-N'-2-ethanesulfonic acid) and 20 mM Glycine at pH-values from 6.5 to 11, and at temperatures from 60 to 100°C.

As shown in Fig. 4, the level of protease activity reached a maximum around pH 9.0. At a pH-value of 5.5 and 11 approximately 10% of activity could be detected. Optimum proteolytic activity for the substrate casein occurred at 95°C. Approximately 90% of enzyme activity was measured at 100°C, and approximately 30% of enzyme activity was measured at 60°C.

**Claims**

1.  A protease, **characterized** by having the following properties:
    (a) pH optimum in the range of from pH 6.0 to 10.0;
    (b) temperature optimum in the range of from 75° to 100°C.
    (c) immunochemical properties identical to those of the protease derived from Thermococcus celer, DSM No. 2476, or Thermococcus sp. AN1, DSM No. 2770, or Thermococcus stetteri, DSM No. 5262, or Thermococcus litoralis, DSM No. 5474.

2.  A protease, **characterized** by having the following properties:
    (a) pH optimum in the range of from pH 6.0 to 10.0;
    (b) temperature optimum in the range of from 75° to 100°C;
    (c) being derived from a strain of the species Thermococcus celer, Thermococcus sp. AN1, Thermococcus stetteri, or Thermococcus litoralis.

3.  A protease according to claim 2, **characterized** by being derived from the strain Thermococcus celer, DSM 2476, or Thermococcus sp. AN1, DSM No. 2770, or Thermococcus stetteri, DSM No. 5262, or Thermococcus litoralis, DSM no. 5474.

4.  A process for the preparation of a protease according to any of claims 1-3, which process comprises cultivation of a protease producing strain of Thermococcus celer, Thermococcus sp. AN1, Thermococcus stetteri, or Thermococcus litoralis in a suitable nutrient medium, containing carbon and nitrogen sources and inorganic salts, followed by recovery of the desired enzyme.

5.  A process according to claim 4, in which Thermococcus celer, DSM No. 2476, Thermococcus sp. AN1, DSM No. 2770, Thermococcus stetteri, DSM No. 5262, or Thermococcus litoralis, DSM No. 5474, or a mutant or a variant thereof, is cultivated.

6.  A proteolytic detergent additive, in the form of a non-dusting granulate, a liquid, in particular a stabilized liquid, a slurry, or a protected enzyme, **characterized** by comprising the protease of any of claims 1-3.

7.  A protease-containing detergent composition, **characterized** by comprising the protease of any of claims 1-3.

8. A detergent composition according to claim 7, which further comprises one or more other enzymes, in particular amylases, lipases, cellulases, or peroxidases.

**Patentansprüche**

1. Eine Protease, dadurch gekennzeichnet, daß sie die folgenden Eigenschaften besitzt:
   (a) pH-Optimum im Bereich von pH 6,0 bis 10,0;
   (b) Temperatur-Optimum im Bereich von 75° bis 100°C;
   (c) immunochemische Eigenschaften, die identisch sind zu denjenigen der Protease, die aus Thermococcus celer, DSM Nr. 2476, oder Thermococcus sp. AN1, DSM Nr. 2770, oder Thermococcus stetteri, DSM Nr. 5262, oder Thermococcus litoralis, DSM Nr. 5474, gewonnen ist.

2. Eine Protease, dadurch gekennzeichnet, daß sie die folgenden Eigenschaften besitzt:
   (a) pH-Optimum im Bereich von pH 6,0 bis 10,0;
   (b) Temperatur-Optimum im Bereich von 75° bis 100°C;
   (c) gewonnen aus einem Stamm der Spezies Thermococcus celer, Thermococcus sp. AN1, Thermococcus stetteri oder Thermococcus litoralis.

3. Eine Protease nach Anspruch 2, dadurch gekennzeichnet, daß sie aus dem Stamm Thermococcus celer, DSM Nr. 2476, oder Thermococcus sp. AN1, DSM Nr. 2770, oder Thermococcus stetteri, DSM Nr. 5262, oder Thermococcus litoralis, DSM Nr. 5474, gewonnen ist.

4. Ein Verfahren zur Herstellung einer Protease nach einem der Ansprüche 1 bis 3, wobei das Verfahren die Kultivation eines Protease-produzierenden Stammes von Thermococcus celer, Thermococcus sp. AN1, Thermococcus stetteri oder Thermococcus litoralis in einem geeigneten Nährstoffmedium, das Kohlenstoff- und Stickstoffquellen und anorganische Salze enthält, umfaßt, gefolgt von der Gewinnung des gewünschten Enzyms.

5. Ein Verfahren nach Anspruch 4, bei dem Thermococcus celer, DSM Nr. 2476, Thermococcus sp. AN1, DSM Nr. 2770, Thermococcus stetteri, DSM Nr. 5262, oder Thermococcus litoralis, DSM Nr. 5474, oder eine Mutante oder eine Variante derselben kultiviert wird.

6. Ein proteolytischer Waschmittelzusatz in der Form eines nicht-staubenden Granulats, einer Flüssigkeit, insbesondere einer stabilisierten Flüssigkeit, einer Aufschlämmung oder eines geschützten Enzyms, dadurch gekennzeichnet, daß er die Protease nach einem der Ansprüche 1 bis 3 umfaßt.

7. Eine proteasehaltige Waschmittelzusammensetzung, dadurch gekennzeichnet, daß sie die Protease nach einem der Ansprüche 1 bis 3 umfaßt.

8. Eine Waschmittelzusammensetzung nach Anspruch 7, die außerdem ein oder mehrere andere Enzyme umfaßt, insbesondere Amylasen, Lipasen, Cellulasen oder Peroxidasen.

**Revendications**

1. Une protéase, caractérisée en ce qu'elle possède les propriétés suivantes :
   a) pH optimal dans la plage de pH 6,0 à 10,0 ;
   b) température optimale dans la plage de 75° à 100°C ;
   c) propriétés immunochimiques identiques à celles de la protéase dérivée de Thermococcus celer, DSM n° 2476, de Thermococcus sp. AN1, DSM n° 5262, de Thermococcus stetteri, DSM n° 5262 ou de Thermococcus litoralis, DSM n° 5474.

2. Une protéase, caractérisée en ce qu'elle possède les propriétés suivantes :
   a) pH optimal dans la plage de 6,0 à 10,0 ;
   b) température optimale dans la plage de 75° à 100°C
   c) provient d'une souche des espèces Thermococcus celer, Thermococcus sp. AN1, Thermococcus stetteri ou Thermococcus litoralis

**3.** Une protéase selon la revendication 2, caractérisée en ce qu'elle provient de la souche Thermococcus celer, DSM 2476, Thermococcus sp. AN1, DSM n° 2770, Thermococcus stetteri, DSM n° 5262 ou Thermococcus litoralis, DSM n° 5474.

**4.** Un procédé de préparation d'une protéase selon l'une des revendications 1 à 3, lequel procédé consiste en la culture d'une souche de Thermococcus celer, de Thermococcus sp. AN1, de Thermococcus stetteri ou de Thermococcus litoralis productrice de protéase dans un milieu nutritif approprié contenant des sources de carbone et d'azote et des sels minéraux, suivie de la récupération de l'enzyme recherchée.

**5.** Un procédé selon la revendication 4, dans lequel on cultive le Thermococcus celer, DSM n° 2476, le Thermococcus sp. AN1, DSM n° 2770, le Thermococcus stetteri, DSM n° 5262 ou le Thermococcus litoralis, DSM n° 5474, un mutant ou une variante de ceux-ci.

**6.** Un additif détergent protéolytique sous la forme de granulés non poudroyants, d'un liquide, en particulier d'un liquide stabilisé, d'une suspension ou d'une enzyme protégée, caractérisé en ce qu'il contient la protéase de l'une des revendicatins 1 à 3.

**7.** Une composition détergente contenant une protéase, caractérisée en ce qu'elle contient la protéase de l'une des revendications 1 à 3.

**8.** Une composition détergente selon la revendication 7 qui contient en outre une ou plusieurs autres enzymes, notamment des amylases, des lipases, des cellulases ou des peroxydases.

Fig. 1

Fig. 2

Fig. 3

Fig. 4